# EUROPÄISCHE PATENTANMELDUNG

(11) **EP 2 294 967 A1**
(43) Veröffentlichungstag der Anmeldung: **16.03.2011**
(21) Anmeldenummer: 10009413.5
(22) Anmeldetag: 10.09.2010
(51) Int. Cl.: A61B 1/008, A61B 1/018

(54) **Endoskopisches Instrument**

(30) Priorität: 14.09.2009 DE 102009041510
(71) Anmelder: Richard Wolf GmbH, 75438 Knittlingen (DE)
(72) Erfinder: Wehrheim, Frank, Dipl.-Ing., 75015 Bretten (DE)
(74) Vertreter: Vollmann, Heiko

(57) **Zusammenfassung**

Ein endoskopisches Instrument weist ein proximalseitig angeordnetes Bedienteil (6), einen distalseitig angeordneten Instrumentenkopf (10) und einen das Bedienteil (6) mit dem Instrumentenkopf (10) verbindenden Schaft (2) auf. An dem Instrumentenkopf (10) münden mehrere durch den Schaft (2) geführte Instrumentenkanäle. Diese Instrumentenkanäle verlaufen in einem an ihre distalen Mündungen angrenzenden Abschnitt bezogen auf eine Mittelachse (A) des Instrumentenkopfes (10) in distaler Richtung schräg nach außen.

## Beschreibung

Die Erfindung betrifft ein endoskopisches Instrument mit den im Oberbegriff des Anspruchs 1 angegebenen Merkmalen.

Instrumente der in Rede stehenden Art werden im medizinischen Bereich eingesetzt und dienen zu Diagnose- und/oder Therapiezwecken im Körperinneren. Solche Instrumente weisen einen Schaft auf, der über natürliche oder künstlich angelegte Kanäle in das Körperinnere geführt wird. An dem distalen Ende des Schaftes ist üblicherweise ein Instrumentenkopf vorgesehen, an dessen distaler Stirnseite Beobachtungs- und Beleuchtungseinrichtungen angeordnet sind. In der Regel mündet an dieser Stirnseite auch mindestens ein Instrumentenkanal für ein durch den Schaft geführtes Hilfsinstrument.

Insbesondere bei chirurgischen Eingriffen, die mit der immer häufiger angewendeten Operationsmethode "NOTES" ("Natural Orifice Transluminal Endoscopic Surgery") durchgeführt werden, ist es erforderlich, mehrere Hilfsinstrumente über den Schaft und den daran angrenzenden Instrumentenkopf zu dem Operationsgebiet zu führen und dort teilweise gleichzeitig zur Verfügung zu stellen. Dementsprechend müssen die hierbei eingesetzten endoskopischen Instrumente mehrere Instrumentenkanäle für Hilfsinstrumente aufweisen, die an dem Instrumentenkopf münden. Häufig wird angestrebt, die an der Stirnseite des Instrumentenkopfes normal zu dieser Stirnseite aus dem Instrument austretenden Hilfsinstrumente zunächst nach außen aufzufächern, um sie anschließend, ähnlich den bei Handhabungsautomaten verwendeten Dreifingergreifern, wieder in unmittelbarer Nähe des Operationsgegenstandes zusammenzuführen. Das Auffächern der Hilfsinstrumente außerhalb des Instrumentes erfordert einen verhältnismäßig großen Raum, so dass dieses Auffächern nur in entsprechend großen Operationsräumen möglich ist. Darüber hinaus steht das Erfordernis mehrerer Instrumentenkanäle nachteilig der bei endoskopischen Instrumenten ansonsten angestrebten Miniaturisierung von Schaft- und Instrumentenkopfquerschnitten entgegen.

Vor diesem Hintergrund liegt der Erfindung die Aufgabe zugrunde, ein endoskopisches Instrument mit mehreren Instrumentenkanälen zu schaffen, das einen vergleichsweise kleinen Schaft- und Instrumentenkopfquerschnitt aufweist und in verhältnismäßig kleinen Operationsräumen bedient und gesteuert werden kann.

Gelöst wird diese Aufgabe durch ein endoskopisches Instrument mit den in Anspruch 1 angegeben Merkmalen. Vorteilhafte Weiterbildungen dieses Instrumentes ergeben sich aus den Unteransprüchen, der nachfolgenden Beschreibung sowie der Zeichnung. Hierbei können gemäß der Erfindung die in den Unteransprüchen und der Beschreibung angegebenen Merkmale jeweils für sich aber auch in Kombination die erfindungsgemäße Lösung gemäß Anspruch 1 weiter ausgestalten.

Das erfindungsgemäße endoskopische Instrument, bei dem es sich bevorzugt um ein medizinisch eingesetztes Instrument handelt, das aber auch ein so genanntes Technoskop zum Einsatz in Hohlräumen von Maschinen und dergleichen sein kann, weist in üblicher Weise ein proximalseitig angeordnetes Bedienteil und einen distalseitig angeordneten Instrumentenkopf auf. Ein Schaft, unter dem im Sinne der Erfindung sowohl ein starrer als auch ein flexibler Hohlschaft zu verstehen ist, wobei auch ein Schlauch ein Schaft sein kann, verbindet das Bedienteil mit dem Instrumentenkopf. Durch den Schaft sind mehrere Instrumentenkanäle geführt. Diese münden an dem Instrumentenkopf. Die Instrumentenkanäle dienen zur Führung von Hilfsinstrumenten zu einem bei Einsatz des endoskopischen Instrumentes außenseitig des distalen Endes des Instrumentenkopfes gelegenen Operationsgebiet.

Gemäß der Erfindung verlaufen die Instrumentenkanäle in einem an ihre distalen Mündungen angrenzenden Abschnitt bezogen auf eine Mittelachse des Instrumentenkopfes in distaler Richtung schräg nach außen. Hierbei sind die schräg zur Mittelachse des Instrumentenkopfes ausgerichteten Abschnitte der Instrumentenkanäle zweckmäßigerweise vollständig in dem Instrumentenkopf angeordnet. Die Mündungen der Instrumentenkanäle sind vorzugsweise nicht im Bereich der Stirnseite des Instrumentenkopfes, sondern an einem daran umfänglich anschließenden Randbereich ausgebildet. Dies ist insofern vorteilhaft als so zum Navigieren des erfindungsgemäßen Instruments an der Stirnseite genügend Raum zur Anordnung einer Beobachtungs- und einer Beleuchtungseinrichtung zur Verfügung steht. Darüber hinaus können an dieser Stirnseite gegebenenfalls auch weitere Einrichtungen, wie z. B. Kraftsensoren zur Erfassung distal wirkender Kräfte sowie Spül- und Absaugeinrichtungen angeordnet sein. Indem aber, anders als bei den bislang bekannten Instrumenten dieser Art, nicht die zur Führung von Hilfsinstrumenten vorgesehenen Instrumentenkanäle an der Stirnseite des Instrumentenkopfes münden, kann der Querschnitt des Instrumentenkopfes typischerweise kleiner als bei den bislang bekannten Instrumenten dimensioniert sein. Ein weiterer Vorteil der nach außen abgewinkelten distalen Endabschnitte der Instrumentenkanäle ist darin zu sehen, dass hierdurch eine gewünschte Auffächerung der Hilfsinstrumente, wie sie oben beschrieben ist, bereits innerhalb des Instrumentenkopfes eingeleitet werden kann. Entsprechend enger können die Hilfsinstrumente um das distale Ende des Instrumentenkopfes herum wieder zusammengeführt werden, so dass für die Auffächerung der Hilfsinstrumente gegenüber den bislang verwendeten endoskopischen Instrumenten deutlich weniger Raum benötigt wird.

Typischerweise handelt es sich bei den in dem erfindungsgemäßen endoskopischen Instrument eingesetzten Hilfsinstrumenten um solche Instrumente, die einen Schaft aufweisen, der zumindest in einem distalen Endabschnitt quer zur Längsausdehnung des Schaftes in mindestens einer Ebene ablenkbar ist. Diese Hilfsinstrumente können in den Instrumentenkanälen besonders leicht geführt werden, wenn die Instrumentenkanäle, wie es eine vorteilhafte Weiterbildung der Erfindung vorsieht, in dem Instrumentenkopf von einem proximalseitigen Abschnitt, der parallel zur Mittelachse des Instrumentenkopfes ausgerichtet ist, bogenförmig in einen distalseitigen, schräg zur Mittelachse des Instrumentenkopfes ausgerichteten Abschnitt übergehen, es also keine durch Abkantungen oder sonst wie gebildete abrupte Richtungsänderungen in den Instrumentenkanälen gibt, an denen die Hilfsinstrumente ansonsten verkanten könnten.

Bevorzugt ist vorgesehen, dass sich der Instrumentenkopf zu einer distalen Stirnfläche hin verjüngt. D. h., der Querschnitt des Instrumentenkopfes nimmt in distaler Richtung bis zum Erreichen einer distalen Stirnseite vorzugsweise kontinuierlich ab. Dementsprechend weist der Instrumentenkopf an seiner distalen Stirnseite seinen geringsten Querschnitt auf, der sich proximalwärts bevorzugt stufenlos zu einem größeren Querschnitt erweitert, wobei dieser größte Querschnitt des Instrumentenkopfes zweckmäßigerweise im Wesentlichen dem Querschnitt des Hohlschaftes entspricht. Ein Vorteil des sich verjüngenden Instrumentenkopfes ist darin zu sehen, dass das erfindungsgemäße endoskopische Instrument über vergleichsweise enge Körperkanäle oder künstlich angelegte Kanäle zu seinem Einsatzgebiet im Körperinneren geführt werden kann, da sich das verjüngende distale Ende des Instrumentenkopfes wie ein Dilatator verhält, der den Zugangskanal im Wesentlichen atraumatisch aufweitet, so dass das endoskopische Instrument mit vergleichsweise geringem Widerstand zu dem Operationsgegenstand geführt werden kann.

Ein weiterer Vorteil des sich distalwärts verjüngenden Instrumentenkopfes zeigt sich dann, wenn die Instrumentenkanäle vorteilhaft umfangseitig an dem sich verjüngenden Abschnitt des Instrumentenkopfes münden. In diesem Fall kann eine Auffächerung der durch die Instrumentenkanäle geführten Hilfsinstrumente bereits innerhalb der lichten Weite des Schaftes bzw. Instrumentenkopfes erfolgen, so dass außerhalb des Instrumentenkopfes noch weniger Raum für die Auffächerung der Hilfsinstrumente benötigt wird.

Der Instrumentenkopf weist vorzugsweise einen Grundkörper auf, an dessen distaler Stirnfläche ein offener Bauraum zur Aufnahme eines Instrumentenmoduls ausgebildet ist. Der offene Bauraum erstreckt sich typischerweise von der Stirnfläche des Grundkörpers in proximaler Richtung. Die Verwendung eines Instrumentenmoduls in dem offenen Bauraum des Instrumentenkopfes ermöglicht es, in einfacher Weise Instrumente herzustellen, die an unterschiedliche Einsatzaufgaben angepasst sind. Für diese unterschiedlichen Aufgaben können verschiedene Konfigurationsvarianten des Instrumentenmoduls bereitgestellt werden, in denen vorteilhaft eine Bilderfassungseinrichtung, eine Beleuchtungseinrichtung, ein Kraftsensor, eine Spüleinrichtung, eine Absaugeinrichtung oder ähnliches in unterschiedlichen Kombinationen oder auch einzeln integriert sein können. Das Instrumentenmodul kann in einfacher Weise in dem offenen Bauraum des Grundkörpers beispielsweise durch Schweißen oder Löten befestigt werden. Bevorzugt ist das Instrumentenmodul allerdings in dem Einbauraum verklebt.

Besonders vorteilhaft ist der Grundkörper des Instrumentenkopfes monolithisch ausgebildet. D. h., der Grundkörper besteht aus einem Stück. Zur

Herstellung des monolithischen Grundkörpers können vorteilhaft Verfahren verwendet werden, die allgemein als "Rapid Prototyping" oder "Rapid Manufacturing" bezeichnet werden. Der Grundkörper kann wahlweise aus einem medizinisch zugelassenen Metall, wie z. B. Edelstahl oder einer Kobalt-Chrom-Legierung, oder aus Kunststoff hergestellt sein. Um den in Folge der in dem Grundkörper abgewinkelten Instrumentenkanäle vergleichsweise komplexen Aufbau des Grundkörpers realisieren zu können, werden metallische Grundkörper bevorzugt schichtweise mittels Lasersinterverfahren oder mittels Verfahren, mit denen der Grundkörperwerkstoff schichtweise mit einem Laser aufgeschmolzen wird, aufgebaut. Grundkörper aus Kunststoffmaterial werden vorzugsweise mittels stereolithografischer Verfahren hergestellt. Mit allen genannten Verfahren kann eine übergangsfreie Qualität der Instrumentenkanäle erzielt werden. Demzufolge werden in den Instrumentenkanälen keine unzugänglichen Ecken oder Hintergreifungen erzeugt, in denen sich ansonsten Ablagerungen und Verunreinigungen sammeln könnten, was im Hinblick auf die im medizinischen Bereich geforderte Hygiene besonders vorteilhaft ist. Darüber hinaus ist die Herstellung des Grundkörpers mit den oben genannten Herstellungsverfahren in der Regel nacharbeitungsfrei und erfolgt ohne zusätzliche aufwändige Montagearbeiten.

Der Austrittswinkel der Instrumentenkanäle an dem Instrumentenkopf bezogen auf die Mittelachse des Instrumentenkopfes liegt bevorzugt in einem Bereich zwischen 5° und 60°. Dieser Winkelbereich ermöglicht die gewünschte Auffächerung der Hilfsinstrumente außerhalb des Instrumentenkopfes, wobei der letztlich gewählte Austrittswinkel der Instrumentenkanäle davon abhängen wird, in welchem Maße die Schäfte der Hilfsinstrumente im Bereich ihres distalen Endes abwinkelbar sind. Die Instrumentenkanäle können an dem Instrumentenkopf alle den gleichen Austrittswinkel oder aber auch unterschiedliche Austrittswinkel aufweisen.

Gemäß einer weiteren vorteilhaften Weiterbildung der Erfindung ist vorgesehen, dass die Instrumentenkanäle an dem Instrumentenkopf gleichmäßig voneinander beabstandet münden. Dementsprechend sind die Mündungen benachbarter Instrumentenkanäle auf die Mittelachse des Instrumentenkopfes bezogen jeweils um einen gleichen Winkelabschnitt versetzt angeordnet. Diese Anordnung ermöglicht einen größtmöglichen Abstand der Instrumentenkanäle zueinander, so dass sich die in benachbarten Instrumentenkanälen geführten Instrumente nicht gegenseitig behindern können, wobei der Abstand der Mündungen benachbarter Instrumentenkanäle aber grundsätzlich von der Anzahl der vorgesehenen Instrumentenkanäle abhängig ist.

Nachfolgend ist die Erfindung anhand von in den Zeichnungen dargestellten Ausführungsbeispielen näher erläutert. In der Zeichnung zeigt:
- Fig. 1: in stark vereinfachter perspektivischer Darstellung ein en- doskopisches Instrument,
- Fig. 2: in stark vereinfachter perspektivischer Darstellung ein zwei- tes endoskopisches Instrument,
- Fig.3: in vergrößerter Darstellung einen Grundkörper eines In- strumentenkopfes eines endoskopischen Instrumentes nach den Fig. 1 und 2 in einer Seitenansicht,
- Fig. 4: den Grundkörper nach Fig. 3 in einer perspektivischen An- sicht,
- Fig. 5: einen Instrumentenkopf eines endoskopischen Instrumen- tes nach den Fig. 1 und 2 in perspektivischer Ansicht,
- Fig. 6: den Grundkörper nach Fig. 3 in einem Längsschnitt,
- Fig. 7: eine separate Ansicht von in dem Instrumentenkopf nach Fig. 5 ausgebildeten Instrumentenkanälen in einem dista- len Endabschnitt,
- Fig. 8: eine separate Ansicht von in dem Instrumentenkopf nach Fig. 5 ausgebildeten Instrumentenkanälen, und

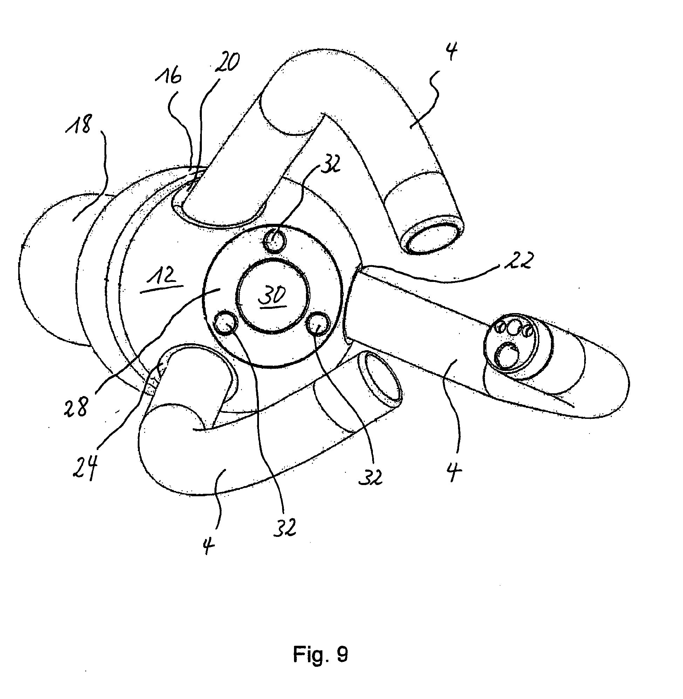

- Fig. 9: in perspektivischer Darstellung den Instrumentenkopf nach Fig. 5 mit an den Mündungen der Instrumentenkanäle aus- tretenden Hilfsinstrumenten.

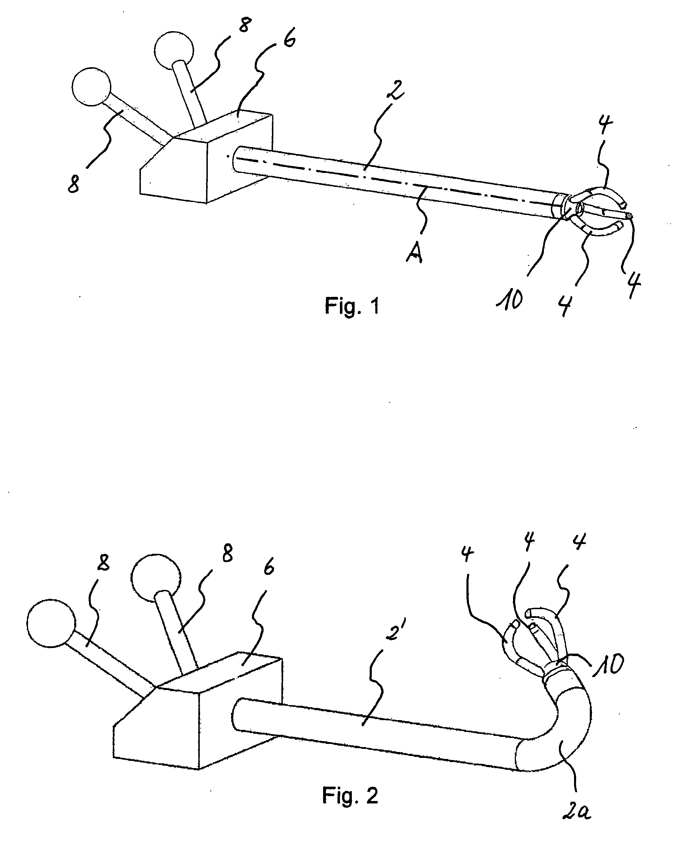

Das in Fig. 1 dargestellte endoskopische Instrument ist ein Hohlschaftinstrument mit einem Schaft 2, der von einem starren Rohr gebildet wird. Der Schaft 2 dient zur Führung von Hilfsinstrumenten 4. Diese Hilfsinstrumente 4 werden durch den Schaft 2 von einem an dem proximalen Ende des Schafts 2 angeordneten Bedienteil 6, an dem sie mit Betätigungshebeln 8 gekoppelt sind, zu einem Instrumentenkopf 10 geführt, der an dem distalen Ende des Schaftes 2 angeordnet ist.

Mit Hilfe der Betätigungshebel 8 können die Hilfsinstrumente 4 in dem endoskopischen Instrument so bewegt werden, dass sie vollständig innerhalb des endoskopischen Instrumentes angeordnet sind, was üblicherweise dann erforderlich ist, wenn das endoskopische Instrument über einen Körperkanal oder einen künstlich angelegten Kanal zu einem Operationsgebiet geführt wird, oder die Hilfsinstrumente 4 können so bewegt werden, dass distale Endabschnitte der Hilfsinstrumente 4, wie in Fig. 1 dargestellt, aus dem Instrumentenkopf 10 des endoskopischen Instrumentes herausragen, was in der Regel im Operationsgebiet erforderlich ist.

An dem Instrumentenkopf 10 werden die Hilfsinstrumente 4 schräg zu einer Mittelachse A von Instrumentenkopf 10 und Schaft 2 aus dem Instrumentenkopf 10 herausgeführt, worauf nachfolgend ausführlicher eingegangen wird. Daher sind die Hilfsinstrumente 4, bei denen es sich ebenfalls um Schaftinstrumente handelt, zumindest in einem distalen Endabschnitt in mindestens einer Ebene quer zu der Mittelachse ihrer Schäfte abwinkelbar ausgebildet. Die Abwinkelbarkeit der Hilfsinstrumente 4 ist derart, dass ihre Enden ähnlich einem Dreifingergreifer an dem Operationsgegenstand wieder zusammengeführt werden können.

Das in Fig. 2 dargestellt endoskopische Instrument unterscheidet sich von dem in Fig. 1 dargestellten Instrument lediglich dahingehend, dass es einen Schaft 2' aufweist, bei dem ein distaler Endabschnitt 2a nicht starr, sondern flexibel ausgebildet ist. Dadurch ist es möglich, den Endabschnitt 2a des Schaftes 2' quer zur Mittelachse des übrigen Schaftes 2' abzuwinkeln bzw. zu krümmen. Ansonsten entspricht das in Fig. 2 gezeigte Instrument dem in Fig. 1 dargestellten Instrument.

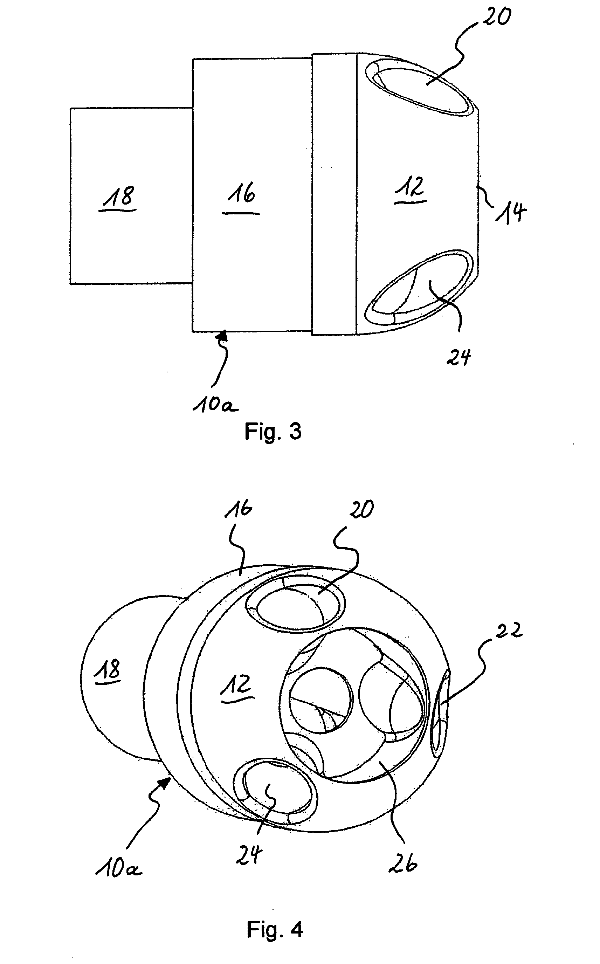

In den Fig. 3, 4 und 6 ist ein Grundkörper 10a eines Instrumentenkopfes 10 dargestellt, der vorzugsweise für den Einsatz in den in den Fig. 1 und 2 dargestellten endoskopischen Instrumenten vorgesehen ist aber auch bei beliebig anderen endoskopischen Instrumenten eingesetzt werden kann. Dieser Grundkörper 10a ist monolithisch ausgebildet und weist drei sich geometrisch unterscheidende Abschnitte auf. Distalseitig ist der Grundkörper 10a in einem ersten Abschnitt 12 kalottenförmig gewölbt ausgebildet, wobei eine Kugelkappe zur Bildung einer distalen Stirnfläche 14 abgeschnitten ist. Demzufolge verringert sich der Außenquerschnitt des Instrumentenkopfes 10 in dem Abschnitt 12 in distaler Richtung kontinuierlich bis auf die Abmessungen der Stirnfläche 14. An den Abschnitt 12 schließt sich proximalseitig ein zylinderförmiger Abschnitt 16 an. Der Abschnitt 16 wird zur Befestigung des Instrumentenkopfes 10 an dem Schaft 2 bzw. 2' in das offene distale Ende des Schaftes 2 bzw. 2' eingeführt. Der Außendurchmesser 16 ist geringfügig kleiner als der größte Außendurchmesser des Abschnitts 12 und entspricht dem Innendurchmesser des Schaftes 2 bzw. 2'. Proximalseitig geht der Abschnitt 16 in einer Stufe in einen zylindrischen Abschnitt 18 über, der einen deutlich geringeren Außendurchmesser als der Abschnitt 16 aufweist und konzentrisch zu dem Abschnitt 16 angeordnet ist.

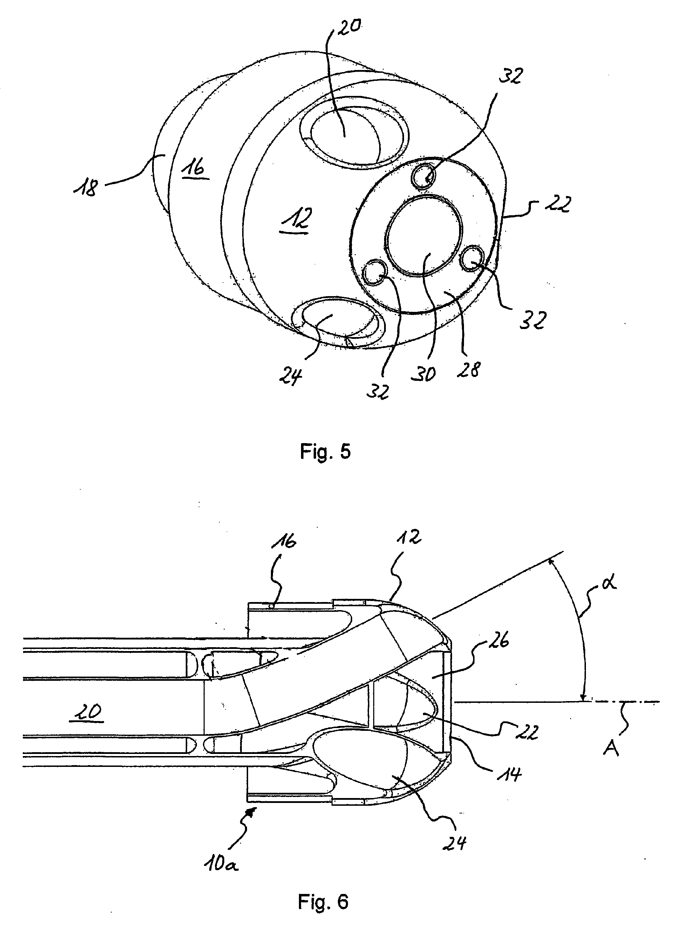

In dem Instrumentenkopf 10 bzw. in dessen Grundkörper 10a sind drei Instrumentenkanäle 20, 22 und 24 ausgebildet. Die Innendurchmesser dieser Instrumentenkanäle 20, 22 und 24 sind bei dem dargestellten Ausführungsbeispiel gleich, können aber prinzipiell auch unterschiedlich sein. In dem Abschnitt 18 des Instrumentenkopfes 10 sind die Instrumentenkanäle 20, 22 und 24 zunächst parallel zu der Mittelachse A des Instrumentenkopfes 10 ausgerichtet, gehen aber bereits in dem Abschnitt 18 bogenförmig in einen Abschnitt über, der bezogen auf die Mittelachse A des Instrumentenkopfes 10 in distaler Richtung in einem Winkel α (Fig. 6) schräg nach außen verläuft. Bei dem in der Zeichnung dargestellten Ausführungsbeispiel beträgt der Winkel α etwa 40°.

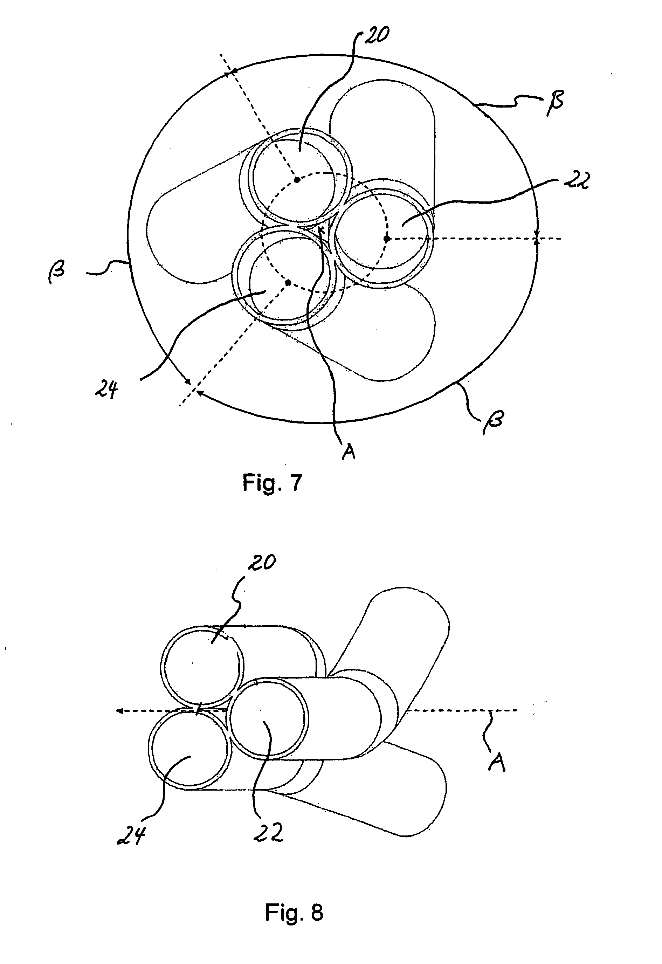

Die Instrumentenkanäle 20, 22 und 24 münden an dem Abschnitt 12 des Grundkörpers 10a in dem gewölbten Bereich, der sich an die Stirnfläche 14 anschließt. Insofern münden die Instrumentenkanäle 20, 22 und 24 in einem Bereich, der innerhalb der lichten Weite des Instrumentenkopfes 10 und des Schaftes 2 bzw. 2' liegt. Die Mündungen der Instrumentenkanäle 20, 22 und 24 sind so über den Umfang des Abschnitts 12 verteilt, dass benachbarte Mündungen jeweils einen gleichen Abstand zueinander aufweisen. Demzufolge schließen die Mittelachsen benachbarter Instrumentenkanäle 20, 22 und 24 bei dem dargestellen Ausführungsbeispiel bezogen auf die Mittelachse A des Instrumentenkopfes 10 jeweils einen Winkel β von 120° ein (Fig. 7).

An der Stirnfläche 14 des distalen Abschnitts 12 des Grundkörpers 10a des Instrumentenkopfes 10 ist eine Ausnehmung 26 ausgebildet. Diese Ausnehmung 26 erstreckt sich konzentrisch zur Mittelachse A des Instrumentenkopfes 10 in proximaler Richtung. Die Ausnehmung 26 bildet einen Einbauraum 26 für ein Instrumentenmodul 28. In dem Instrumentenmodul 28 sind eine Beobachtungseinrichtung 30 und drei Beleuchtungseinrichtungen 32 integriert, wie sie aus dem Stand der Technik bereits bekannt sind. Das Instrumentenmodul 28 ist in dem Grundkörper 10a des Instrumentenkopfes 10 durch eine Klebverbindung derart stoffschlüssig befestigt, dass die Stirnseite des Instrumentenmoduls 28 mit der Stirnfläche 14 des Grundkörpers 10a fluchtet.

Mit den Beleuchtungseinrichtungen 32 wird das in distaler Verlängerung der Mittelachse A des Instrumentenkopfes 10 gelegene Operationsgebiet ausgeleuchtet und mit der Beobachtungseinrichtung 30 betrachtet. Dabei verhindert die Anordnung der Instrumentenkanäle 20, 22 und 24 radial außenseitig des Instrumentenmoduls 28 und die schräge Ausrichtung der Instrumentenkanäle 20, 22 und 24, dass das Operationsgebiet von den durch die Instrumentenkanäle 20, 22 und 24 geführten Hilfsinstrumente 4 abgeschattet wird, da die Hilfsinstrumente 4 zunächst um das Beleuchtungs- und Beobachtungsfeld außenseitig herum bzw. in einem außenseitigen Randbereich des Beleuchtungs- und Beobachtungsfelds geführt werden und erst in unmittelbarer Nähe des Operationsgegenstandes wieder zusammengeführt werden.

### Bezugszeichenliste

- 2, 2': Schaft
- 2a: Endabschnitt
- 4: Hilfsinstrument
- 6: Bedienteil
- 8: Betätigungshebel
- 10: Instrumentenkopf
- 10a: Grundkörper
- 12: Abschnitt
- 14: Stirnfläche
- 16: Abschnitt
- 18: Abschnitt
- 20: Instrumentenkanal
- 22: Instrumentenkanal
- 24: Instrumentenkanal
- 26: Ausnehmung, Einbauraum
- 28: Instrumentenmodul
- 30: Beobachtungseinrichtung
- 32: Beleuchtungseinrichtung
- A: Mittelachse
- α: Winkel
- β: Winkel

## Patentansprüche

1. Endoskopisches Instrument mit einem proximalseitig angeordneten Bedienteil (6), einem distalseitig angeordneten Instrumentenkopf (10) und mit einem das Bedienteil (6) mit dem Instrumentenkopf (10) verbindenden Schaft (2, 2'), wobei an dem Instrumentenkopf (10) mehrere durch den Schaft (2, 2') geführte Instrumentenkanäle (20, 22, 24) münden, **dadurch gekennzeichnet, dass** die Instrumentenkanäle (20, 22, 24) in einem an ihre distalen Mündungen angrenzenden Abschnitt bezogen auf eine Mittelachse (A) des Instrumentenkopfes (10) in distaler Richtung schräg nach außen verlaufen.

2. Endoskopisches Instrument nach Anspruch 1, **dadurch gekennzeichnet, dass** die Instrumentenkanäle (20, 22, 24) in dem Instrumentenkopf (10) von einem proximalseitigen Abschnitt, der parallel zur Mittelachse (A) des Instrumentenkopfes (10) ausgerichtet ist, bogenförmig in einen distalseitigen, schräg zur Mittelachse (A) des Instrumentenkopfes (10) ausgerichteten Abschnitt übergehen.

3. Endoskopisches Instrument nach einem der vorangehenden Ansprüche, **dadurch gekennzeichnet, dass** sich der Instrumentenkopf (10) zu einer distalen Stirnfläche (14) verjüngt.

4. Endoskopisches Instrument nach Anspruch 3, **dadurch gekennzeichnet, dass** die Instrumentenkanäle (20, 22, 24) umfangsseitig an dem sich verjüngenden Abschnitt (12) des Instrumentenkopfes (10) münden.

5. Endoskopisches Instrument nach einem der vorangehenden Ansprüche, **dadurch gekennzeichnet, dass** der Instrumentenkopf (10) einen Grundkörper (10a) aufweist, an dessen distaler Stirnfläche (14) ein offener Bauraum (26) zur Aufnahme eines Instrumentenmoduls (28) ausgebildet ist.

6. Endoskopisches Instrument nach Anspruch 5, **dadurch gekennzeichnet, dass** das Instrumentenmodul (28) in dem Einbauraum (26) verklebt ist.

7. Endoskopisches Instrument nach einem der Ansprüche 5 oder 6, **dadurch gekennzeichnet, dass** in das Instrumentenmodul (28) eine Beobachtungs- (30) und/oder Beleuchtungseinrichtung (32) integriert ist.

8. Endoskopisches Instrument nach einem der Ansprüche 5 bis 7, **dadurch gekennzeichnet, dass** der Grundkörper (10a) des Instrumentenkopfes (10) monolithisch ausgebildet ist.

9. Endoskopisches Instrument nach einem der vorangehenden Ansprüche, **dadurch gekennzeichnet, dass** der Austrittswinkel (α) der Instrumentenkanäle (20, 22, 24) an dem Instrumentenkopf (10) bezogen auf die Mittelachse (A) des Instrumentenkopfes (10) in einem Bereich zwischen 5° und 60° liegt.

10. Endoskopisches Instrument nach einem der vorangehenden Ansprüche, **dadurch gekennzeichnet, dass** die Instrumentenkanäle (20, 22, 24) an dem Instrumentenkopf (10) gleichmäßig voneinander beabstandet münden.
